# EUROPEAN PATENT APPLICATION

(11) **EP 1 860 196 A1**
(43) Date of publication of application: **28.11.2007**
(21) Application number: 06714938.5
(22) Date of filing: 22.02.2006
(51) Int. Cl.: C12Q 1/02, C12N 5/02

(54) **METHOD OF ANALYZING DYNAMICS IN THE DIFFERENTIATION OF VASCULAR ENDOTHELIAL PROGENITOR CELLS**

(30) Priority: 23.02.2005 JP 2005047422
(71) Applicant: Foundation for Biomedical Research and Innovation, Kobe-shi, Hyogo 6500047 (JP); TOKAI UNIVERSITY EDUCATIONAL SYSTEM, Shibuya-ku Tokyo 1510063 (JP)
(72) Inventor: ASAHARA, T. Institute of Biomed. Res. & Innovation, Chuo-ku, Kobe-shi, Hyogo 6500047 (JP); MASUDA, Haruchika, Isehara-shi, Kanagawa, 2591193 (JP)
(74) Representative: Duckworth, Timothy John
(86) International application number: PCT/JP2006/303817
(87) International publication number: WO 2006/090886

(57) **Abstract**

The present invention provides a method of forming an EPC colony with good reproducibility, and a method of analyzing the dynamics of EPC differentiation in the body of patient. More specifically, the present invention provides a method of analyzing the dynamics of differentiation of endothelial progenitor cells, which includes culturing hemangioblasts in a semisolid medium containing a vascular endothelial growth factor and a basic fibroblast growth factor, and evaluating the mode of endothelial progenitor cell colony formation; a method of forming an endothelial progenitor cell colony which includes culturing hemangioblasts in a semisolid medium containing a vascular endothelial growth factor and a basic fibroblast growth factor; a semisolid medium containing a vascular endothelial growth factor and a basic fibroblast growth factor; and a kit for preparing the semisolid medium and the like.

## Description

### Technical Field

The present invention relates to a method of analyzing dynamics in the differentiation of endothelial progenitor cells, a kit therefor and the like.

### Background Art

Life style-related diseases such as diabetes, hypertension, hyperlipidemia, obesity and the like are risk factors of vascular disorders, and finally cause organ failure such as arteriosclerosis obliterans (ASO), myocardial infarction, renal failure and the like. The number of patients afflicted with these lifestyle-related diseases is extremely large, and it may be no exaggeration to say that the treatment of blood vessel is the most important task for the modern medical care. For example, in arteriosclerosis obliterans which causes leg gangrene, the below-knee amputation is often unavoidable in many cases. While the tissues and organs that fell into such ischemic state have been treated with vasodilation by catheter, surgical revascularization using intravenous graft and the like, they have not become effective methods for severely affected patients.

In recent years, angiogenic therapy has been used as a new treatment method. The angiogenic therapy is largely divided into gene therapy and cell transplantation method. With regard to the gene therapy, a reported method comprises injecting a plasmid of vascular endothelial growth factor (VEGF) or hepatocyte growth factor (HGF) into an ischemic lesion to improve the blood flow. On the other hand, in the cell transplantation method, bone marrow-derived mononuclear cell transplantation therapy and peripheral blood mobilized CD34 vascular stem cell transplantation therapy by administration of granulocyte colony stimulating factor (G-CSF) are clinically applied as a method for transplanting an endothelial progenitor cell (hereinafter to be also abbreviated as EPC) capable of differentiating into vascular endothelial cell. However, a method of evaluating the dynamics of EPC differentiation in peripheral blood used for comprehending the pathology of patients and treatment effect has not been established as yet.

In a cell transplantation method, in general, it is necessary to know in advance the EPC-forming ability of a cell suspension used for transplantation (the cell suspension contains cells capable of differentiating into EPC) so as to achieve efficient angiogenesis by EPC in the ischemic site or efficient cell amplification in vitro. Moreover, analysis of dynamics of EPC differentiation in the body of a patient after cell transplantation is important for grasping the pathology of patient and treatment effect. At present, a method comprising use of a methylcellulose medium is employed (Atsushi Hirao, Yoichi Takaue et al., Journal of Clinical Apheresis 10: 17-22 (1999)) for determining the colony-forming ability of hematopoietic stem cell capable of differentiating into red blood cell, T-lymphocyte, B-lymphocyte, monocyte/macrophage, granulocyte, megakaryocyte and the like. However, there is no known method for assaying the colony-forming ability of EPC.

### Disclosure of the Invention

An object of the present invention is to provide a method capable of analyzing dynamics of EPC differentiation in the body of a patient, a kit therefor and the like.

In view of the above-mentioned problems, the present inventors have studied cultivation conditions permitting EPC to form a colony, and succeeded in forming an EPC colony with high reproducibility by the use of a semisolid medium containing a particular physiologically active substance. Moreover, the present inventors have found that two kinds of, namely, large and small colonies in different levels of differentiation are formed during the colony formation, and the dynamics of EPC differentiation in the body of a patient can be analyzed by tracking the expression thereof, which resulted in the completion of the present invention.

Accordingly, the present invention provides the following:
[1] a method of analyzing dynamics of differentiation of an endothelial progenitor cell, which comprises culturing a hemangioblast in a semisolid medium containing a vascular endothelial growth factor and a basic fibroblast growth factor, and evaluating the mode of vascular endothelial progenitor cell colony formation;
[2] the method of the above-mentioned [1], wherein the hemangioblast is derived from bone marrow, cord blood or peripheral blood;
[3] the method of the above-mentioned [1], wherein the hemangioblast is a mononuclear cell;
[4] the method of the above-mentioned [1], wherein the hemangioblast is CD34 positive and/or CD133 positive;
[5] the method of the above-mentioned [1], wherein the hemangioblast is mobilized by a substance capable of mobilizing a hemangioblast;
[6] the method of the above-mentioned [5], wherein the substance capable of mobilizing a hemangioblast is a granulocyte colony stimulating factor;
[7] the method of the above-mentioned [1], wherein the hemangioblast is derived from human;
[8] the method of the above-mentioned [1], wherein the semisolid medium is selected from the group consisting of a methylcellulose medium, a matrigel, a collagen gel and a Mebiol gel;
[9] the method of the above-mentioned [1], wherein the semisolid medium further contains one or more factors selected from the group consisting of a stem cell factor, interleukin 3, an insulin-like growth factor and an epithelial cell growth factor;
[10] the method of the above-mentioned [9], wherein the semisolid medium further contains a serum and/or heparin;
[11] a method of forming an endothelial progenitor cell colony, which comprises culturing a hemangioblast in a semisolid medium containing a vascular endothelial growth factor and a basic fibroblast growth factor and confirming the appearance of an endothelial progenitor cell colony;
[12] a semisolid medium containing a vascular endothelial growth factor and a basic fibroblast growth factor;
[13] a kit for preparing a semisolid medium containing a vascular endothelial growth factor and a basic fibroblast growth factor, which comprises a vascular endothelial growth factor, a basic fibroblast growth factor and a semisolid medium (one or both of the vascular endothelial growth factor and the basic fibroblast growth factor has/have not been added to the semisolid medium).

The analysis method of dynamics of EPC differentiation of the present invention enables more efficient EPC transplantation therapy. That is, an EPC colony assay can be conducted along with a blood lineage cell colony assay of hemangioblast to be transplanted to a patient, which enables prediction and comprehension of the treatment effect. In addition, the pathology of patients can be known by determining the state of expression of EPC colonies in different levels of differentiation.

### Brief Description of the Drawings

Fig. 1 shows the outline of the EPC colony assay method using a methylcellulose medium. The case where a hemangioblast to be a sample is a cord blood-derived mononuclear cell is shown as one example.
Fig. 2 shows the observation results of endothelial cell-like small cell colony and endothelial cell-like large cell colony using a phase-contrast microscope, which appeared in the EPC colony assay process in the present invention. The case where a hemangioblast to be a sample is a cord blood-derived mononuclear cell is shown as one example. Cord blood-derived mononuclear cells were seeded on the methylcellulose medium of the present invention and two kinds of EPC colonies having different cell sizes appeared after culturing for 14 - 18 days.
Fig. 3 shows the results of double-staining of EPC colony derived from cord blood CD133 positive cell with acLDL-DiI and UEA-1 lectin-FITC. a, c and e each show a large cell colony, b, d and f each show a small cell colony, a and b each show a phase-contrast image, c and d each show an image stained with acLDL-DiI, and e and f each show an image stained with UEA-1 lectin-FITC. Both colonies were stained with acLDL-DiI and UEA-1 lectin-FITC, and exhibited a feature of EPC.
Fig. 4 shows the time schedule of EPC colony assay using peripheral blood of ASO patients to whom CD34 positive cell, which had been mobilized by G-CSF, was transplanted.
Fig. 5 shows the outline of EPC colony assay method using a methylcellulose medium. The case where a hemangioblast to be a sample is a peripheral blood-derived mononuclear cell is shown as one example.
Fig. 6 shows the number of mononuclear cells and CD34 positive cells in peripheral blood on administration of G-CSF for the purpose of mobilizing CD34 cells in peripheral blood, and on transplantation of CD34 positive cell in ASO patient (case 1).
MNC: mononuclear cell, CD34⁺: CD34 positive

Fig. 7 shows the EPC colony-forming ability (small cell colony, large cell colony) of peripheral blood on administration of G-CSF for the purpose of mobilizing CD34 cells in peripheral blood, and on transplantation of CD34 positive cell in ASO patient (case 1).
MNC: mononuclear cell, CFU: colony-forming unit, CD34+Tx: CD34⁺ cell transplantation

Fig. 8 shows the number of mononuclear cells and CD34 positive cells in peripheral blood on administration of G-CSF for the purpose of mobilizing CD34 cells in peripheral blood, and on transplantation of CD34 positive cell in ASO patient (case 2).
MNC: mononuclear cell, CD34⁺: CD34 positive, CD34+Tx: CD34⁺ cell transplantation

Fig. 9 shows the EPC colony-forming ability (small cell colony, large cell colony) of peripheral blood on administration of G-CSF for the purpose of mobilizing CD34 cells in peripheral blood, and on transplantation of CD34 positive cell in ASO patient (case 2).
MNC: mononuclear cell, CFU: colony-forming unit, CD34+Tx: CD34⁺ cell transplantation

Fig. 10 shows the number of mononuclear cells and CD34 positive cells in peripheral blood on administration of G-CSF for the purpose of mobilizing CD34 cells in peripheral blood, and on transplantation of CD34 positive cell in ASO patient (case 3). MNC: mononuclear cell, CD34⁺: CD34 positive, CD34+Tx: CD34⁺ cell transplantation

Fig. 11 shows the EPC colony-forming ability (small cell colony, large cell colony) of peripheral blood on administration of G-CSF for the purpose of mobilizing CD34 cells in peripheral blood, and on transplantation of CD34 positive cell in ASO patient (case 3).
MNC: mononuclear cell, CFU: colony-forming unit, CD34+Tx: CD34⁺ cell transplantation

### Best Mode for Embodying the Invention

The present invention provides a method of analyzing the dynamics of differentiation of an endothelial progenitor cell. The analysis method of the present invention comprises, for example, culturing a hemangioblast in a semisolid medium containing a vascular endothelial growth factor and/or a basic fibroblast growth factor, and evaluating the mode of endothelial progenitor cell colony formation.

The "hemangioblast" used in the present invention refers to a progenitor cell of both blood lineage cell and vascular endothelial cell, and is not particularly limited as long as it is an undifferentiated cell, which can be a blood cell (e.g., red blood cell, T-lymphocyte, B-lymphocyte, monocyte/macrophage, granulocyte, megakaryocyte) via a blood stem cell or blood progenitor cell, or can be a vascular endothelial cell via EPC. As the hemangioblast, a cell derived from bone marrow, cord blood or peripheral blood of a test subject can be used. The hemangioblast may also be a mononuclear cell.

Moreover, the hemangioblast may be CD34 and/or CD133 positive. Accordingly, as the hemangioblast, use of only preliminarily selected CD34 positive and/or CD133 positive cells is also preferable. For selection of CD34 positive and/or CD133 positive cells, cell sorting methods generally used in the art are used. Specifically, magnetic cell sorting method (MACS), fluorescent cell sorting method (FACS) and the like, which use a substance having specific affinity to CD34 antigen and/or CD133 antigen, can be mentioned.

As the substance having specific affinity to CD34 antigen or CD133 antigen, for example, an antibody having specific affinity to these proteins or a fragment thereof can be mentioned. The specific affinity means the ability to specifically recognize and bind to the protein by antigen-antibody reaction. The antibody or fragment thereof is not particularly limited as long as it can specifically bind to the protein. It may be a polyclonal antibody, monoclonal antibody or functional fragment thereof. These antibody or functional fragment thereof can be produced by methods generally used in the art. For example, when a polyclonal antibody is used, a method comprising injecting the protein subcutaneously on the back, intraperitoneally, intravenously or the like to an animal such as mouse and rabbit to immunize the animal and, after increase of the antibody titer, collecting the antiserum can be mentioned. When a monoclonal antibody is used, a method comprising producing hybridoma according to a conventional method and collecting a secretory fluid thereof can be mentioned. As a method of producing an antibody fragment, a method comprising expression of a cloned antibody gene fragment in a microorganism and the like is frequently used. The purity of the antibody, antibody fragment and the like is not particularly limited as long as it maintains specific affinity to the protein. The antibody and fragment thereof may be labeled with a fluorescent substance, enzyme, radioisotope or the like.

The hemangioblast may also be one mobilized by a substance capable of mobilizing a hemangioblast. As the substance capable of mobilizing a hemangioblast, for example, G-CSF, SDF-1, estrogen, VEGF, GM-CSF, angiopoietin 1 and 2, HGF, statins and erythropoietin can be mentioned. Of these, G-CSF is preferable. In this case, the hemangioblast may be provided in the form of a body fluid component (e.g., peripheral blood) containing mobilized hemangioblasts, which was obtained by administration of a substance capable of mobilizing a hemangioblast to a test subject. The frequency, period and dose of administration of a substance capable of mobilizing a hemangioblast to a test subject also vary depending on the kind of the substance to be used, and is not particularly limited as long as it can mobilize a hemangioblast. For G-CSF, the frequency of administration is, for example, generally one to three times a day, preferably twice, the administration period is, for example, about 3 - 7 days, preferably about 4 - 6 days, more preferably 5 days, and the dose is about 2 - 50 µg/kg/day, preferably about 5 - 20 µg/kg/day, more preferably about 10 µg/kg/day.

The hemangioblast may also be one collected from a test subject, to whom a hemangioblast mobilized by a substance capable of mobilizing a hemangioblast was transplanted.

The test subject in the present invention means a mammal in general including human, which needs to be analyzed for dynamics of EPC differentiation. In view of the object of the present invention, i.e., clinical application, however, the test subject is preferably human. The use in a mammal such as mouse, rat, dog, cat, rabbit, bovine, swine, goat, sheep, horse, monkey and the like is also preferable.

While the semisolid medium to be used in the present invention is not particularly limited as long as it enables formation of an endothelial progenitor cell colony, for example, a semisolid medium known as a medium and the like for growing an undifferentiated cell such as hematopoietic stem cell and the like can be used. As such semisolid medium, for example, a methylcellulose medium, a matrigel, a collagen gel, a Mebiol gel and the like can be mentioned. When a methylcellulose medium is used as the semisolid medium, the concentration of methylcellulose in the medium is not particularly limited. For example, the concentration is about 0.5 - 5%, preferably about 0.7 - 3%, more preferably about 1%.

The vascular endothelial growth factor (VEGF) to be used in the present invention is a growth factor specifically acting on EPC, and known to be mainly produced in a perivascular cell. Several kinds of VEGF proteins having different sizes are produced by selective splicing. The VEGF to be used in the present invention may be any type of VEGF as long as it enables colony formation of EPC. It is preferably VEGF₁₆₅. While the derivation and the like of VEGF are not particularly limited, a recombinant expected to ensure a stable supply is preferable, and a human recombinant is particularly preferable. Commercially available ones are known. The concentration of VEGF in the semisolid medium varies depending on the kind of VEGF to be used, and is not particularly limited as long as it is appropriate for formation of an endothelial progenitor cell colony. When human recombinant VEGF₁₆₅ is used, the concentration is, for example, about 5 - 500 ng/mL, preferably about 20 - 100 ng/mL, more preferably about 50 ng/mL.

The basic fibroblast growth factor (bFGF) to be used in the present invention is about 18 kDa of single-strand peptide with 9.0 of isoelectric point, and known to have functions such as angiogenesis, stimulation of growth of various cells, differentiation induction and the like. While the derivation and the like of bFGF to be used in the present invention are not particularly limited, a recombinant expected to ensure a stable supply is preferable, and a human recombinant is particularly preferable. Commercially available ones are known. The concentration of bFGF in the semisolid medium varies depending on the kind of bFGF to be used, and is not particularly limited as long as it is appropriate for formation of an endothelial progenitor cell colony. When human recombinant bFGF is used, the concentration is, for example, about 5 - 500 ng/mL, preferably about 20 - 100 ng/mL, more preferably about 50 ng/mL.

In the view of more efficient formation of an endothelial progenitor cell colony, the semisolid medium to be used in the present invention preferably further contains one or more factors, preferably not less than 3 factors, more preferably all factors, selected from the group consisting of stem cell factor (SCF), interleukin 3 (IL-3), insulin-like growth factor (IGF) and epithelial cell growth factor (EGF), in addition to the above-mentioned VEGF and/or bFGF. Accordingly, the semisolid medium to be used in the method of the present invention may be a medium containing, for example, a) SCF, b) IL-3, c) IGF, d) EGF, e) a combination of SCF and IL-3, f) a combination of SCF and IGF, g) a combination of SCF and EGF, h) a combination of IL-3 and IGF, i) a combination of IL-3 and EGF, j) a combination of IGF and EGF, k) a combination of SCF, IL-3 and IGF, 1) a combination of SCF, IL-3 and EGF, m) a combination of SCF, IGF and EGF, n) a combination of IL-3, IGF and EGF, or o) a combination of SCF, IL-3, IGF and EGF, in addition to VEGF and/or bFGF.

The stem cell factor (SCF) is a glycoprotein with about 30,000 of molecular weight, which consists of 248 amino acids. While there exist a soluble form and a membrane-bound form due to alternative splicing, SCF to be used in the present invention may be of any form, as long as it enables colony formation of EPC. It is preferably of a soluble form. While the derivation and the like of SCF are not particularly limited, a recombinant expected to ensure a stable supply is preferable, and a human recombinant is particularly preferable. Commercially available ones are known. The concentration of SCF in the semisolid medium varies depending on the kind of SCF to be used, and is not particularly limited as long as it enables more efficient formation of endothelial progenitor cell colony. When human recombinant SCF is used, the concentration is, for example, 10 - 1000 ng/mL, preferably 50 - 500 ng/mL, more preferably about 100 ng/mL.

The interleukin 3 (IL-3) is known as a cytokine that acts on a hematopoietic stem cell and various hematocyte lineage progenitor cells to promote proliferation and differentiation thereof. While it consists of 152 residues and 166 residues of amino acids for human and mouse, respectively, it has 28,000 of apparent molecular weight due to the modification of sugar chains. While IL-3 to be used in the present invention is appropriately selected according to the derivation of EPC whose colony is to be formed, when it is used for colony formation of human EPC, human IL-3 is preferable, and a recombinant expected to ensure a stable supply is particularly preferable. Commercially available ones are known. The concentration of IL-3 in the semisolid medium varies depending on the kind of IL-3 to be used, and is not particularly limited as long as it enables more efficient formation of an endothelial progenitor cell colony. When human recombinant IL-3 is used, the concentration is, for example, about 1 - 500 ng/mL, preferably about 5 - 100 ng/mL, more preferably about 20 ng/mL.

The insulin-like growth factor (IGF) is also referred to as somatomedin, which is a polypeptide with about 7,000 of molecular weight having a primary structure similar to that of proinsulin. It is known that there are two types, i.e., IGF-I and IGF-II, similar to each other. Both are known to have similar action and promote growth of various cells in vitro. IGF to be used in the present invention may be of any type, as long as it enables colony formation of EPC. It is preferably IGF-I. While the derivation and the like of IGF are not particularly limited, a recombinant expected to ensure a stable supply is preferable, and a human recombinant is particularly preferable. Commercially available ones are known. The concentration of IGF in the semisolid medium varies depending on the kind of IGF to be used, and is not particularly limited as long as it enable more efficient formation of endothelial progenitor cell colony. When human recombinant IGF-I is used, the concentration is about 5 - 500 ng/mL, preferably about 20 - 100 ng/mL, more preferably about 50 ng/mL.

The epithelial cell growth factor (EGF) is a protein consisting of 53 amino acids, which has the action of promoting the differentiation and growth of epithelial cells, and is known to be unbound with a sugar chain. It is about 6 kDa and has disulfide bonds at three sites. While the derivation and the like of EGF to be used in the present invention are not particularly limited, a recombinant expected to ensure a stable supply is preferable, and a human recombinant is particularly preferable. Commercially available ones are known. The concentration of EGF in the semisolid medium varies depending on the kind of EGF to be used, and is not particularly limited as long as it enables more efficient formation of an endothelial progenitor cell colony. When human recombinant EGF is used, the concentration is about 5 - 500 ng/mL, preferably about 20 - 100 ng/mL, more preferably about 50 ng/mL.

From the aspect of more efficient formation of an endothelial progenitor cell colony, the semisolid medium to be used in the present invention preferably further contains a serum and/or heparin, in addition to the above-mentioned VEGF and bFGF, and one or more factors selected from the group consisting of SCF, IL-3, IGF and EGF.

When a serum is used, the derivation and the like thereof are not particularly limited. Since it is added to a medium, the amount thereof to be used is expected to be relatively large. Thus, commercially available sera from bovine, horse, human and the like (e.g., fetal serum) are used. It is more preferably fetal calf serum (FCS). The serum is preferably used after inactivation. The concentration of serum in the semisolid medium varies depending on the kind of serum to be used, and is not particularly limited as long as it enables more efficient formation of an endothelial progenitor cell colony. When FCS is used, the concentration is about 10 - 50%, preferably about 15 - 40%, more preferably about 30%.

Heparin is a glucosaminoglycan having a repeat structure of disaccharide consisting of an uronic acid residue comprising one of D-glucuronic acid and L-iduronic acid and a D-glucosamine as the skeleton. A large number of heparins are present in the small intestine and the lung of mammals. Many commercially available products are extracts from swine bowel, and the molecular weights thereof are about 7,000 - 25,000. The heparin to be used in the present invention may be derived from an animal tissue, and may be a chemically or physically decomposed low molecular heparin, as long as it enables colony formation of EPC. For example, a heparin from swine bowel is used. Commercially available ones are known. The concentration of heparin in the semisolid medium varies depending on the kind of heparin to be used, and is not particularly limited as long as it enables more efficient formation of an endothelial progenitor cell colony. When a heparin from swine bowel is used, the concentration is about 0.2 - 10 U/mL, preferably about 1 - 5 U/mL, more preferably about 2 U/mL.

The semisolid medium, which can be most preferably used in the present invention, is a semisolid medium containing about 50 ng/mL of vascular endothelial growth factor, about 50 ng/mL of basic fibroblast growth factor and about 100 ng/mL of stem cell factor, about 20 ng/mL of interleukin 3, about 50 ng/mL of epithelial cell growth factor, about 50 ng/mL of insulin-like growth factor and about 30% of serum, and about 2 U/mL of heparin.

Each of the above-mentioned physiologically active substances is dissolved in a semisolid medium to a given concentration, or a concentrated solution of each physiologically active substance (stock solution) is prepared in advance and diluted with a semisolid medium to a given concentration, whereby the semisolid medium of the present invention to be used for the analysis of dynamics of EPC differentiation can be prepared. For example, the physiologically active substance-containing semisolid medium of the present invention can be prepared by dissolving the necessary physiologically active substances in a commercially available semisolid medium to given concentrations and sterilizing the medium by filtration and the like, or aseptically adding the stock solutions sterilized by filtration and the like to a commercially available semisolid medium to dilute them. Sterilization by filtration can be performed according to a method generally employed in the art. For example, it is performed using 0.22 µm or 0.45 µm of Millipore filter and the like.

A hemangioblast can be cultured in a semisolid medium containing the aforementioned physiologically active substances by adding a cell suspension containing the hemangioblast to the semisolid medium containing the aforementioned physiologically active substances. As the cell suspension, a body fluid itself containing a hemangioblast (e.g., bone marrow aspirate, cord blood, peripheral blood) can also be used. The cultivation conditions for a hemangioblast are not particularly limited as long as it permits colony formation, and those generally employed in the art can be utilized. The cultivation is performed generally under a 5% CO₂ atmosphere at 37°C generally for not less than 10 days, for example, for 14 - 18 days or longer. While formation of colony can be visually confirmed, whether or not the obtained colony indeed consists of EPC is determined, for example, by confirming the ability of acetylated LDL (acLDL) uptake, bindability with UEA-1 lectin, expression of VE-cadherin, KDR, vWF (e.g., by RT-PCR or fluorescence immunohistochemical analysis) and the like. For example, when a colony is double-stained with DiI-labeled acetylated LDL (acLDL-DiI) and FITC-labeled UEA-1 lectin (UEA-1 lectin-FITC), the colony is double-stained in the case of EPC.

The mode of EPC colony formation can be evaluated according to the size of cells forming the colony. As mentioned in the Examples below, when EPC colony is formed in the present invention, two kinds of EPC colonies having different sizes appear. These two kinds of EPC colonies having different sizes appear when generally not less than 10 days, for example, 14 - 18 days, have passed after seeding a hemangioblast on a semisolid medium. Therefore, the cells are cultivated, for example, for 14 - 18 days before formation of an EPC colony in the present invention. Of the two kinds of colonies to be formed, a colony consisting of large cells (hereinafter also referred to as endothelial cell-like large cell colony; CFU-Large cell like EC, large cell colony) mainly includes cells with about 20 - 50 µm of diameter, and a colony consisting of small cells (hereinafter also referred to as endothelial cell-like small cell colony; CFU-small cell like EC, small cell colony) mainly includes cells with about 20 µm or below of diameter (e.g., about 10 - 20 µm). The "mainly" used herein means that about 30%, preferably about 50%, particularly preferably about 70%, of the cell population constituting the colony are cells with about 20 - 50 µm of diameter (for large cell colony) or cells with 20 µm or below of diameter (for small cell colony). It has been clarified that when bone marrow fluid, cord blood or peripheral blood and the like are collected from a test subject over time and the collected samples are subjected to EPC colony assay, the time necessary for a colony after mobilization to appear varies between the large cell colony and the small cell colony. The large cell colony appears somewhat later than the small cell colony. Accordingly, the presence of EPCs in different differentiation stages is suggested. An endothelial cell-like small cell that appears in an early stage is an EPC in an early differentiation stage, and an endothelial cell-like large cell that appears in a later stage is an EPC in a late differentiation stage. According to the analysis method of the present invention, EPCs in different levels of differentiation can be distinguished based on the difference in the size of cells forming a colony as mentioned above. Therefore, the dynamics of EPC differentiation can be analyzed.

The present invention also provides a method of forming an endothelial progenitor cell colony, which comprises culturing a hemangioblast in a semisolid medium containing a vascular endothelial growth factor and/or a basic fibroblast growth factor. The colony-forming method of the present invention can further comprise confirmation of the appearance of an endothelial progenitor cell colony.

The present invention further provides a semisolid medium containing the aforementioned factors, and a reagent for the analysis of dynamics of the differentiation of endothelial progenitor cells, comprising the semisolid medium.

The present invention also provides a kit comprising VEGF, bFGF and a semisolid medium. The kit of the present invention contains one or both of VEGF and bFGF in a form isolated from the semisolid medium (e.g., stored in a different container). As such kit, for example, an embodiment wherein each of a) VEGF, bFGF and a semisolid medium is stored in an independent container, b) a semisolid medium containing VEGF and bFGF are stored in independent containers, or c) a semisolid medium containing bFGF and VEGF are stored in independent containers, can be mentioned. In addition, one or more factors selected from the group consisting of SCF, IL-3, IGF and EGF, as well as serum and/or heparin may be provided in a form isolated from or added to the semisolid medium. The kit of the present invention can be useful, for example, for preparing the semisolid medium of the present invention.

The kit of the present invention may further contain at least one element selected from the group consisting of a substance capable of mobilizing a hemangioblast and a substance having specific affinity to the cell surface marker of a hemangioblast. The substance capable of mobilizing a hemangioblast and the cell surface marker of a hemangioblast are as mentioned above. Such kit can be preferably used for the analysis method of the present invention.

The present invention is explained in more detail in the following by referring to the Examples, which are described for the explanation of the present invention and do not limit the present invention in any way.

### Examples

### Example 1: EPC colony assay method using methylcellulose medium (cord blood)

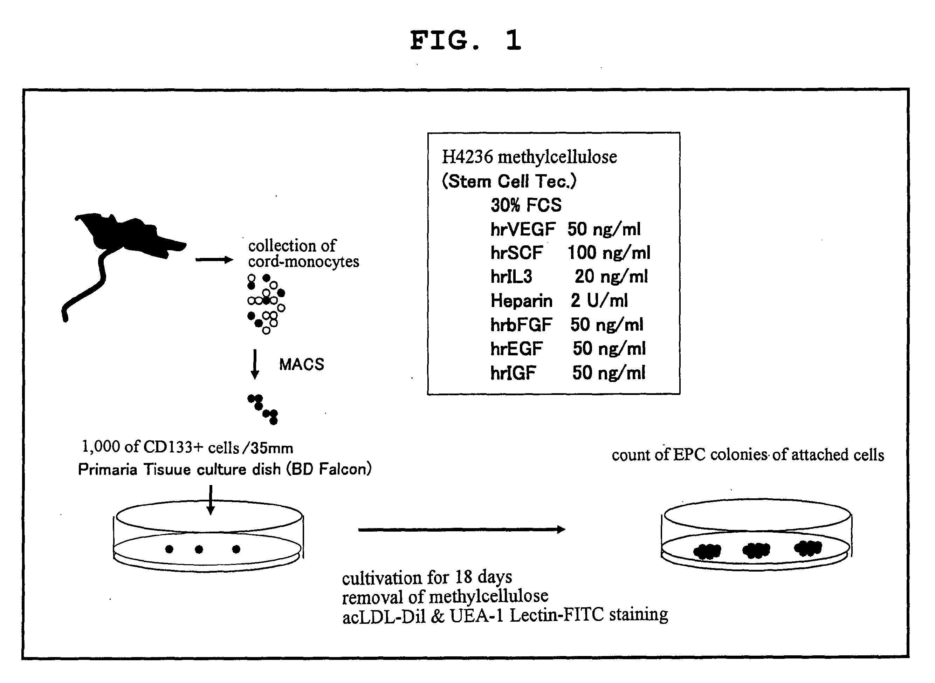

The experiment protocol is shown in Fig. 1.

### (1) Preparation of methylcellulose medium containing physiologically active substance

The methylcellulose medium containing physiologically active substance was prepared according to the composition shown in Table 1 and using a methylcellulose medium (H4236, Stem Cell Tec.). To be specific, each of the components shown in Table 1 was aseptically added to a methylcellulose medium to a given concentration.

**Table 1**

| component | provided by | concentration |
|---|---|---|
| FCS | JRH (cat no. 12303-500M) | 30% |
| hrVEGF | Peprotec | 50 ng/mL |
| hrSCF | Kirin Brewery | 100 ng/mL |
| hrlL-3 | Kirin Brewery | 20 ng/mL |
| heparin | Ajinomoto Pharma | 2 U/mL |
| hrbFGF | Peprotec | 50 ng/mL |
| hrEGF | Peprotec | 50 ng/mL |
| hrlGF | Peprotec | 50 ng/mL |

In Table 1, "h" shows human-derived, and "r" shows a recombinant produced gene-engineeringly. Other abbreviations are as mentioned above.

### (2) EPC colony assay

As the cell suspension containing hemangioblastm, a cell suspension containing cord blood-derived mononuclear cells was used. First, the collected blood was overlaid on Histopaque-1077, and mononuclear cells were separated by density-gradient centrifugation. The separated mononuclear cells were washed with PBS-EDTA. The platelet was removed, and the mononuclear cells were collected and suspended in buffer to give a cell suspension.

Then, the cell suspension was subjected to MACS using anti-CD133 antibody and CD133 positive cells were recovered. For detail, a CD133 positive cell isolation kit (manufactured by Militenyi Biotec, catalog No.130-050-801) was used, and the protocol of the package insert was followed.

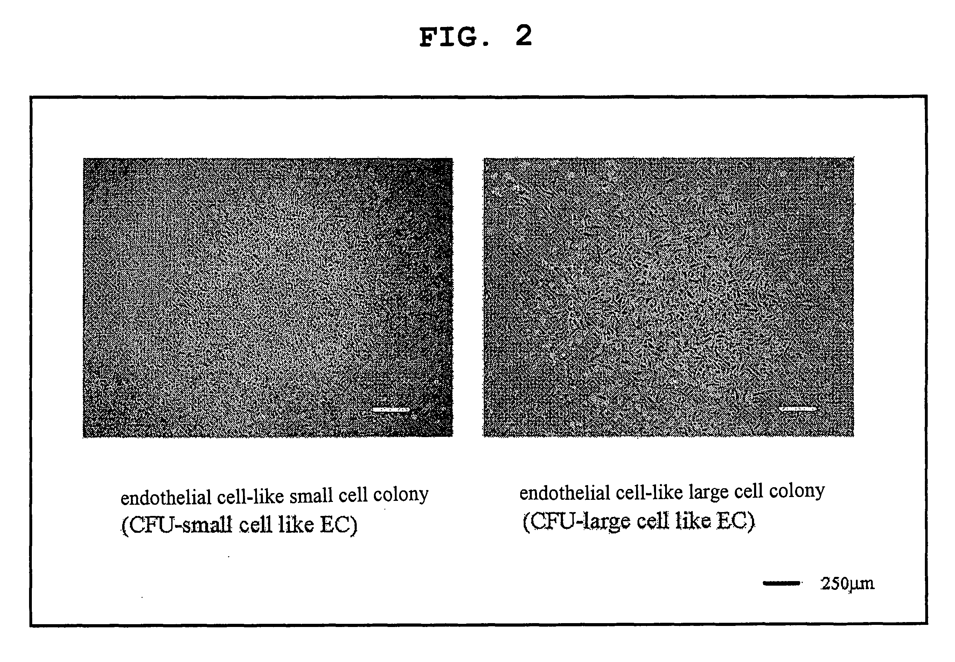

The obtained CD133 positive cells were seeded on Primaria Tissue Culture dish (BD Falcon) with 35 mm of diameter at the concentration of 1,000 cells per 1 mL of the physiologically active substance-containing methylcellulose medium produced in the above-mentioned (1), and cultured at 37°C for 18 days in the presence of 5% CO₂. Then, the methylcellulose medium was removed, and the non-adherent cells were washed away with PBS. The colony of the cells attached to the culture dish was observed to find that two kinds of EPC colonies having different individual cell sizes had appeared. The colony of small cells is conveniently referred to as endothelial cell small cell colony (CFU-small cell like EC), and the colony of large cells is conveniently referred to as endothelial cell large cell colony (CFU-large cell like EC). Fig. 2 is an image of each colony observed with a phase contrast microscope. These colonies were double-stained with acLDL-DiI and UEA-1 lectin-FITC. For detail, after removal of methylcellulose, 1 ml of EGM-MV-added EBM-2 (5% FCS medium) (Clonetics Co., Single quots kit) was added. Then, acLDL-DiI (10 µl) was added, and the cells were cultured for 3 hr. After washing twice with PBS, the above-mentioned medium and FITC labeled-UEA1 lectin (manufactured by Sigma) were added to the medium to a concentration of 0.2 µg/ml, and the medium was cultured for 3 hr. After washing twice, the medium was exchanged and the cells were observed with a fluorescence microscope.

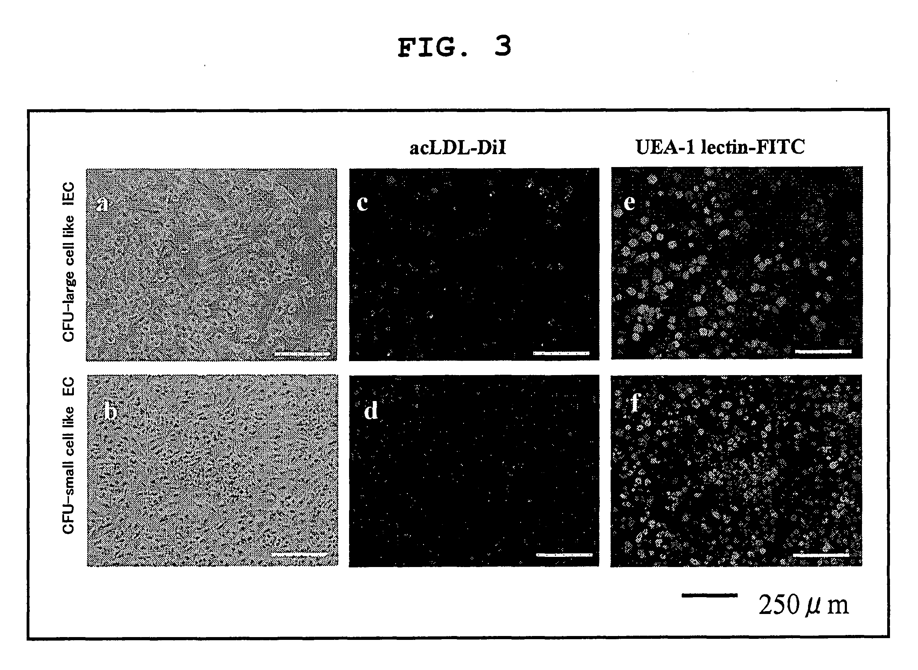

The results are shown in Fig. 3. Both colonies were stained with acLDL-DiI and UEA-1 lectin-FITC, and exhibited the characteristics of EPC.

The small cell EPC colonies that appeared in the methylcellulose medium were collected and cultured in a medium for cultivation of endothelial cells (EGM-MV-added EBM-2 (5% FCS medium)) at 37°C for 36 hr in the presence of 5% CO₂. After cultivation, the expression of VE cadherin or KDR antigen, which is a cell surface antigen of endothelial cells, and CD45 antigen, which is a cell surface antigen of hematocyte lineage cells, was examined. As a result, the cells expressing VE cadherin or KDR antigen were observed.

### Example 2: EPC colony assay method using methylcellulose medium (peripheral blood)

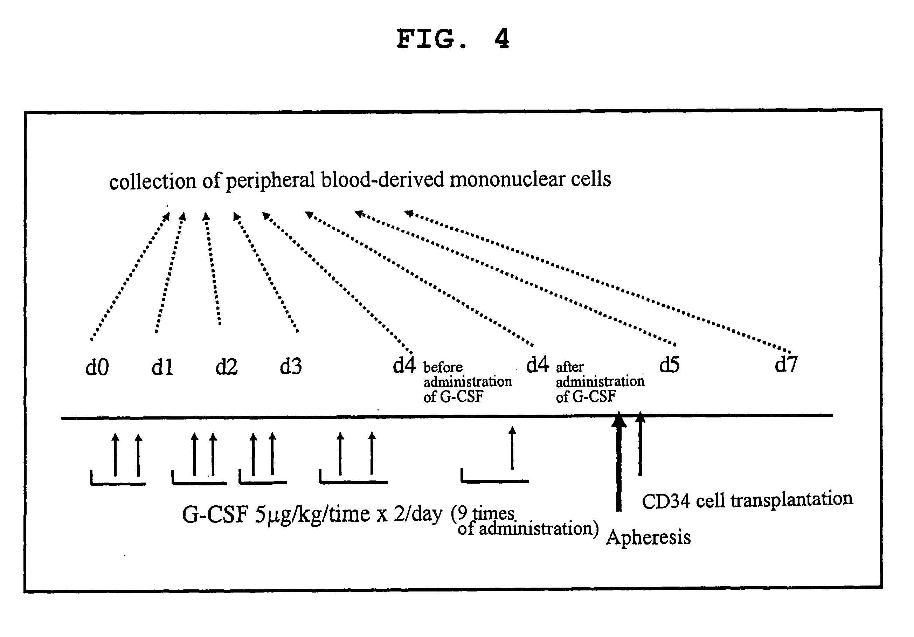

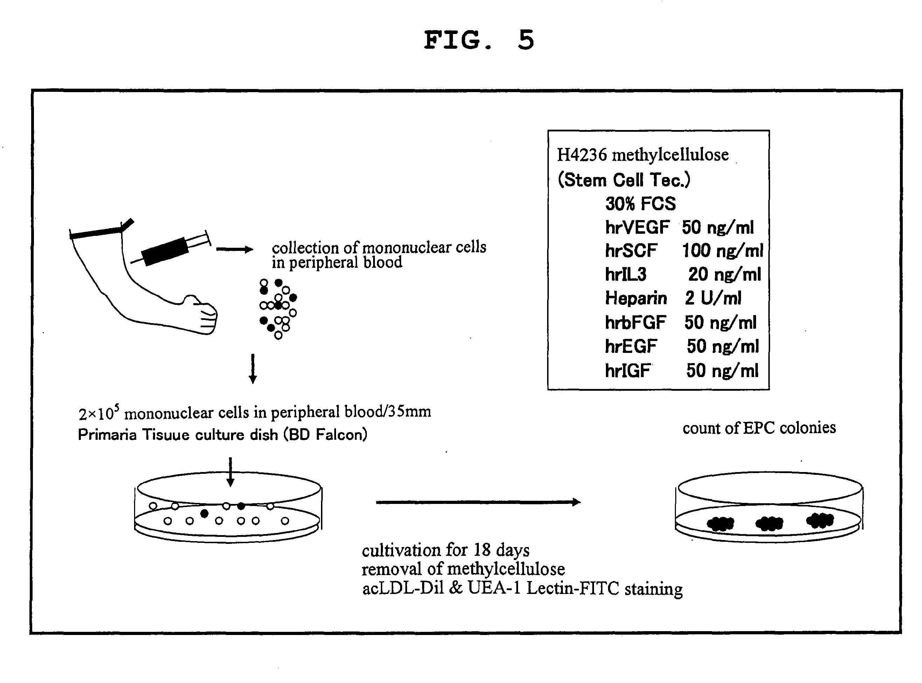

With ASO patients (3 cases), to whom CD34 positive cell mobilized by G-CSF had been transplanted, as subjects, the dynamics of EPC on administration of G-CSF aiming to mobilize CD34 cells in peripheral blood and on cell transplantation was evaluated using the EPC colony assay method of the present invention. The experiment protocols are shown in Fig. 4 and Fig. 5. As the physiologically active substance-containing methylcellulose medium, a medium same as the one prepared in Example 1 was used.

In this Example, as the cell suspension containing hemangioblastm, the peripheral blood (including mononuclear cell) collected from ASO patient, to whom CD34 positive cell mobilized by G-CSF had been transplanted as mentioned above, was used. The peripheral blood was not subjected to cell sorting by MACS, and the cell suspension was directly applied to the assay. For mobilizing by G-CSF, G-CSF (manufactured by Kirin Brewery Co., Ltd.) was subcutaneously administered twice daily each at a dose of 5 µg/kg body weight of patient. G-CSF was administered for 5 days (administered only once on the fifth day).

Peripheral blood was collected from the patients over time, and mononuclear cells were separated. The obtained peripheral blood-derived mononuclear cells were seeded on Primaria Tissue Culture dish (BD Falcon) with 35 mm of diameter at a concentration of 2×10⁵ cells per 1 mL of the physiologically active substance-containing methylcellulose medium, and cultured at 37°C for 18 days in the presence of 5% CO₂. Then, the methylcellulose medium was removed; and the non-adherent cells were washed away with PBS. The colony of the cells attached to the culture dish was observed to find that two kinds of EPC colonies having different sizes had appeared as in Example 1. In the same manner as in Example 1, each colony was double-stained with acLDL-DiI and UEA-1 lectin-FITC to find that both were double-stained.

In the following, the results of the number of mononuclear cells and CD34 positive cells in peripheral blood and the EPC colony-forming ability (small cell colony, large cell colony) on administration of G-CSF aiming to mobilize CD34 cells in peripheral blood and on transplantation of CD34 positive cell are shown for each case.

The number of mononuclear cells and CD34 positive cells was determined by placing the cell suspension in a hemocytometer to visually count under an optical microscope. The EPC colony-forming ability was determined according to the EPC colony-forming method and the method for evaluating colony-forming ability of the present invention.

### Case 1 (78 years of age, male)

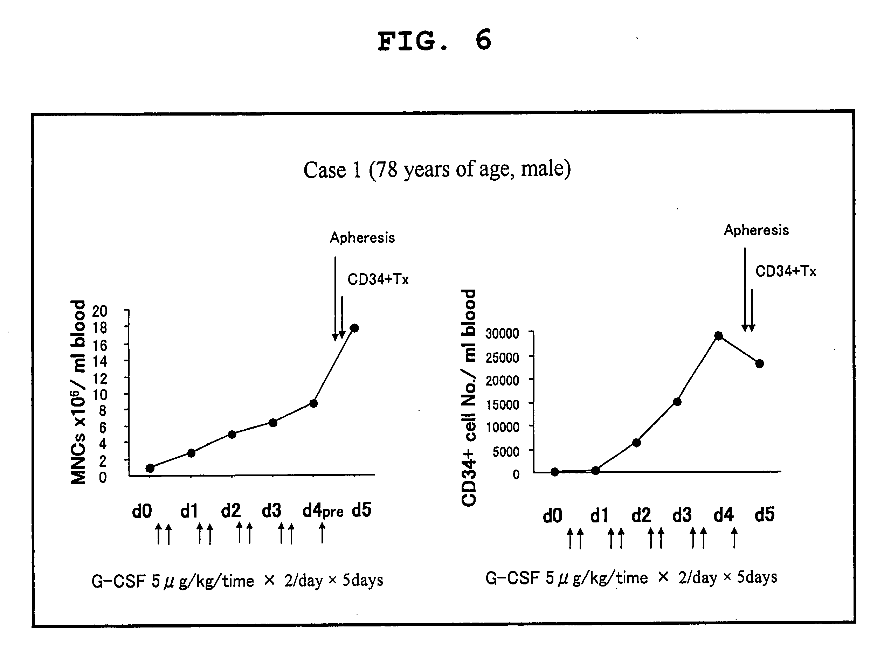

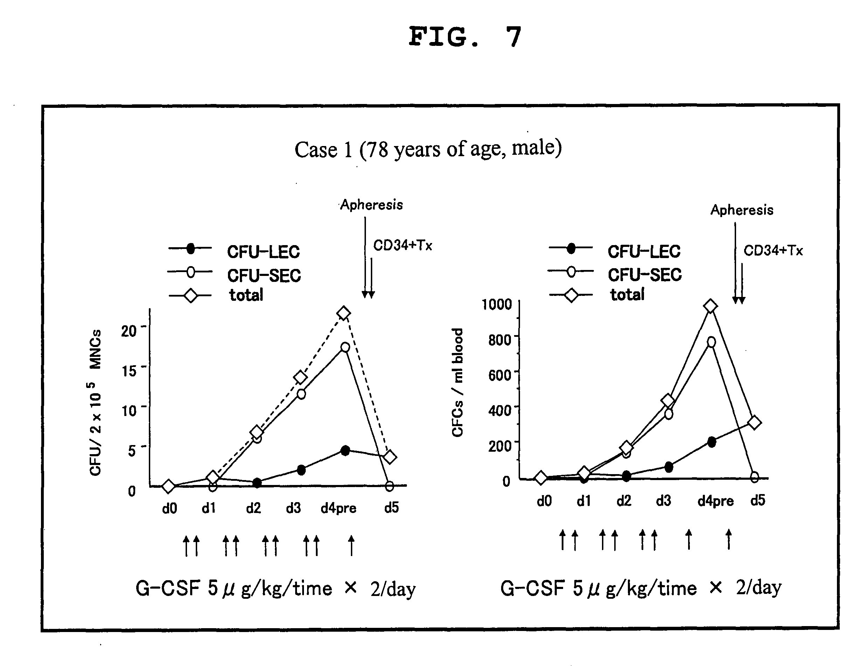

The number of mononuclear cells per 1 mL of peripheral blood and the number of CD34 positive cells per 1 mL of peripheral blood are shown in Fig. 6. The number of colonies formed when 2×10⁵ of peripheral blood mononuclear cells were subjected to the EPC colony assay of the present invention, and the number of colonies formed per 1 mL of peripheral blood were respectively measured for each of small cell colonies, large cell colonies and total EPC colonies, and the results are shown in Fig. 7.

In case 1, the peripheral blood mononuclear cells and CD34 positive cells increased due to the administration of G-CSF. In addition, the EPC colony-forming ability per 2×10⁵ of peripheral blood mononuclear cells and per 1 ml of peripheral blood increased due to the administration of G-CSF.

### Case 2 (72 years of age, male)

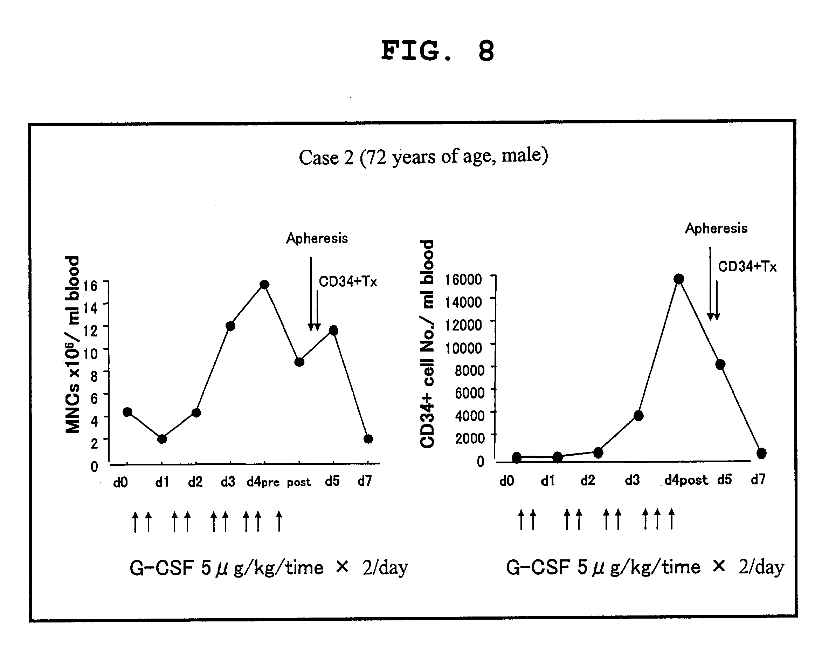

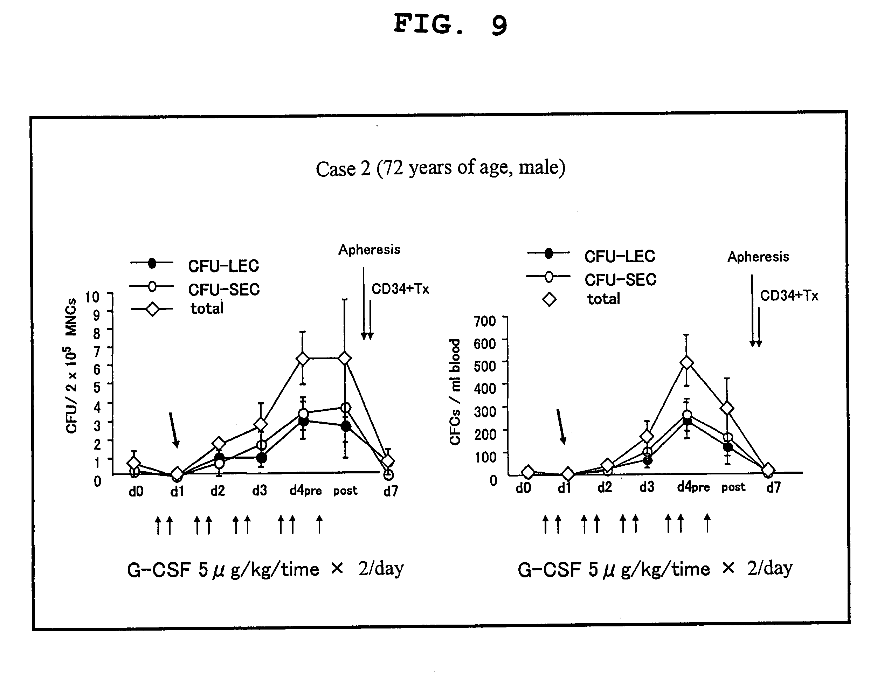

The number of mononuclear cells per 1 mL of peripheral blood and the number of CD34 positive cells per 1 mL of peripheral blood are shown in Fig. 8. The number of colonies formed when 2×10⁵ of peripheral blood mononuclear cells were subjected to the EPC colony assay of the present invention, and the number of colonies formed per 1 mL of peripheral blood were respectively measured for each of small cell colonies, large cell colonies and total EPC colonies, and the results are shown in Fig. 9.

Also in case 2, the peripheral blood mononuclear cells and CD34 positive cells increased due to the administration of G-CSF. In addition, the EPC colony-forming ability per 2×10⁵ of peripheral blood mononuclear cells and per 1 ml of peripheral blood increased due to the administration of G-CSF.

### Case 3 (68 years of age, male)

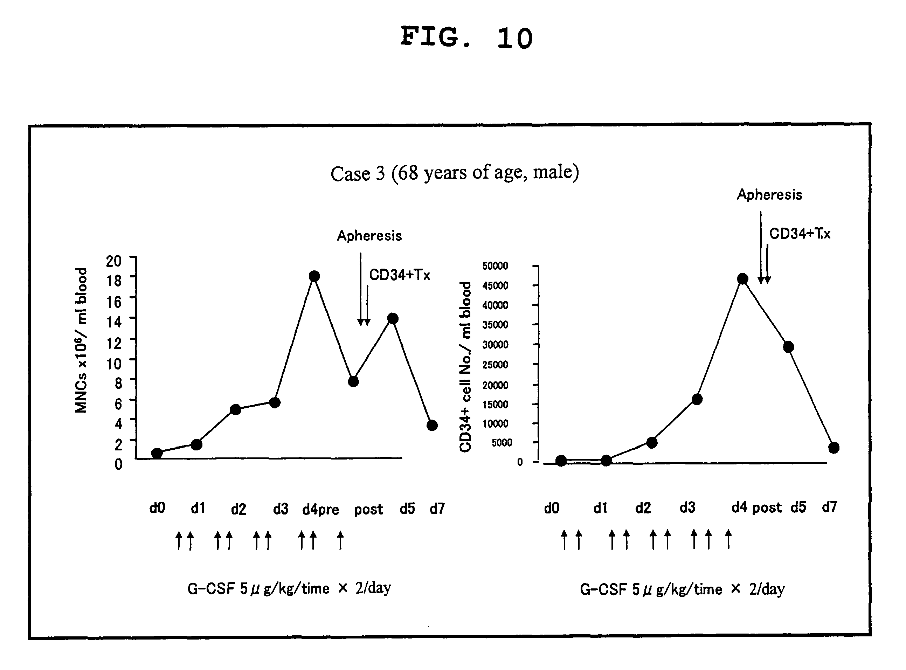

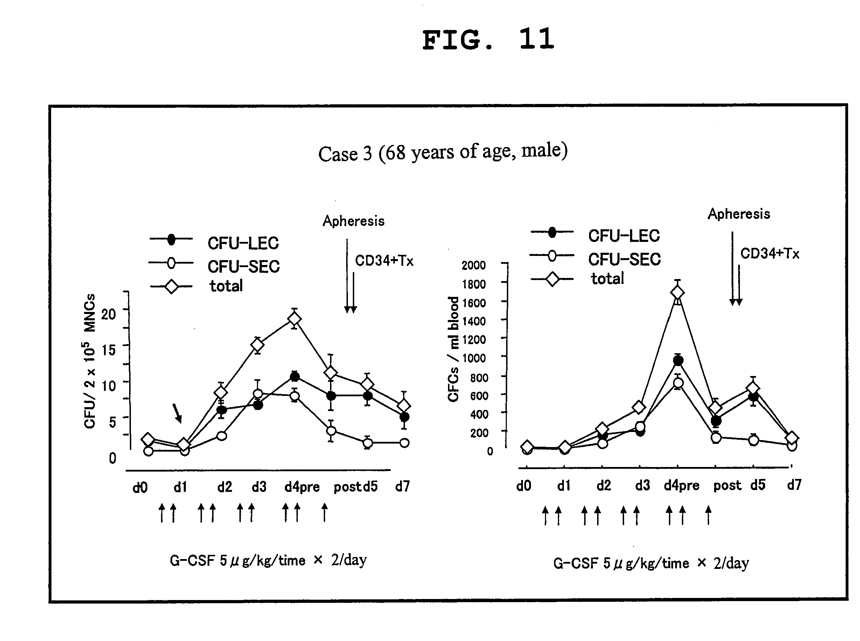

The number of mononuclear cells per 1 mL of peripheral blood and the number of CD34 positive cells per 1 mL of peripheral blood are shown in Fig. 10. The number of colonies formed when 2×10⁵ of peripheral blood mononuclear cells were subjected to the EPC colony assay of the present invention, and the number of colonies formed per 1 mL of peripheral blood were respectively measured for each of small cell colonies, large cell colonies and total EPC colonies, and the results are shown in Fig. 11.

Also in case 3, the peripheral blood mononuclear cells and CD34 positive cells increased due to the administration of G-CSF. In addition, the EPC colony-forming ability per 2×10⁵ of peripheral blood mononuclear cells and per 1 ml of peripheral blood increased due to the administration of G-CSF.

From the above results, it has been clarified that the EPC colony assay method of the present invention is useful for clinically comprehension of the dynamics of EPC differentiation in peripheral blood of patient.

### Industrial Applicability

The analysis method of the dynamics of EPC differentiation of the present invention enables more efficient EPC transplantation therapy. That is, an EPC colony assay can be performed along with a blood lineage cell colony assay of a hemangioblast to be transplanted to patient, which in turn enables prediction and comprehension of the treatment effect. In addition, the pathology of patient can be known by determining the state of expression of EPC colonies in different levels of differentiation.

This application is based on a patent application No. 2005-047422 filed in Japan (filing date: February 23, 2005), the contents of which are incorporated in full herein by this reference.

## Claims

1. A method of analyzing dynamics of differentiation of an endothelial progenitor cell, which comprises culturing a hemangioblast in a semisolid medium containing a vascular endothelial growth factor and a basic fibroblast growth factor, and evaluating the mode of vascular endothelial progenitor cell colony formation.

2. The method of claim 1, wherein the hemangioblast is derived from bone marrow, cord blood or peripheral blood.

3. The method of claim 1, wherein the hemangioblast is a mononuclear cell.

4. The method of claim 1, wherein the hemangioblast is CD34 positive and/or CD133 positive.

5. The method of claim 1, wherein the hemangioblast is mobilized by a substance capable of mobilizing a hemangioblast.

6. The method of claim 5, wherein the substance capable of mobilizing a hemangioblast is a granulocyte colony stimulating factor.

7. The method of claim 1, wherein the hemangioblast is derived from human.

8. The method of claim 1, wherein the semisolid medium is selected from the group consisting of a methylcellulose medium, a matrigel, a collagen gel and a Mebiol gel.

9. The method of claim 1, wherein the semisolid medium further contains one or more factors selected from the group consisting of a stem cell factor, interleukin 3, an insulin-like growth factor and an epithelial cell growth factor.

10. The method of claim 9, wherein the semisolid medium further contains a serum and/or heparin.

11. A method of forming an endothelial progenitor cell colony, which comprises culturing a hemangioblast in a semisolid medium containing a vascular endothelial growth factor and a basic fibroblast growth factor and confirming the appearance of an endothelial progenitor cell colony.

12. A semisolid medium containing a vascular endothelial growth factor and a basic fibroblast growth factor.

13. A kit for preparing a semisolid medium containing a vascular endothelial growth factor and a basic fibroblast growth factor, which comprises a vascular endothelial growth factor, a basic fibroblast growth factor and a semisolid medium (one or both of the vascular endothelial growth factor and the basic fibroblast growth factor has/have not been added to the semisolid medium).
